# EUROPÄISCHE PATENTSCHRIFT

(11) **EP 1 988 828 B1**
(45) Veröffentlichungstag und Bekanntmachung des Hinweises auf die Patenterteilung: **19.06.2013**
(21) Anmeldenummer: 07704535.9
(22) Anmeldetag: 12.02.2007
(51) Int. Cl.: A61B 5/0478, A61N 1/05

(54) **SONDE ZUR DATENÜBERTRAGUNG ZWISCHEN EINEM GEHIRN UND EINER DATENVERARBEITUNGSVORRICHTUNG**
PROBE FOR DATA TRANSMISSION BETWEEN A BRAIN AND A DATA PROCESSING DEVICE
SONDE DE TRANSMISSION DE DONNÉES ENTRE UN CERVEAU ET UN DISPOSITIF DE TRAITEMENT DE DONNÉES

(30) Priorität: 23.02.2006 DE 102006008501
(43) Veröffentlichungstag der Anmeldung: 12.11.2008
(73) Patentinhaber: CorTec GmbH, 79110 Freiburg (DE)
(72) Erfinder: RICKERT, Jörn, 79117 Freiburg (DE); MEHRING, Carsten, 79106 Freiburg (DE); BALL, Tonio, 79117 Freiburg (DE); AERTSEN, Ad, 79102 Freiburg (DE); SCHULZE-BONHAGE, Andreas, 79102 Freiburg (DE)
(74) Vertreter: Schneider, Günther Martin
(86) Internationale Anmeldenummer: PCT/EP2007/051359
(87) Internationale Veröffentlichungsnummer: WO 2007/096268

(56) Entgegenhaltungen:
- WO-A1-00/09008
- US-A- 4 735 208
- US-A1- 2004 082 984
- TAKAHASHI HIROKAZU ET AL: "Microelectrode array on folding polyimide ribbon for epidural mapping of functional evoked potentials." IEEE TRANSACTIONS ON BIO-MEDICAL ENGINEERING APR 2003, Bd. 50, Nr. 4, April 2003 (2003-04), Seiten 510-516, XP002442777 ISSN: 0018-9294
- SCHERBERGER H ET AL: "Magnetic resonance image-guided implantation of chronic recording electrodes in the macaque intraparietal sulcus." JOURNAL OF NEUROSCIENCE METHODS 30 NOV 2003, Bd. 130, Nr. 1, 30. November 2003 (2003-11-30), Seiten 1-8, XP002442778 ISSN: 0165-0270
- OHL F W ET AL: "Topographic analysis of epidural pure-tone-evoked potentials in gerbil auditory cortex." JOURNAL OF NEUROPHYSIOLOGY MAY 2000, Bd. 83, Nr. 5, Mai 2000 (2000-05), Seiten 3123-3132, XP002442779 ISSN: 0022-3077

## Beschreibung

Die Erfindung betrifft eine Sonde zur Datenübertragung zwischen einem Gehirn und einer Datenverarbeitungsvorichtung, sowie ein Herstellungsverfahren für eine derartige Sonde. Die Erfindung betrifft ferner ein Verfahren zur Datenübertragung zwischen einem Gehirn eines Lebewesens und einer Datenverarbeitungsvorrichtung.

Bei der Messung neuronaler Aktivität im Gehirn eines lebenden Tieres oder des Menschen ergibt sich das Problem, dass räumlich und zeitlich gut aufgelöste Aktivitätsmuster nur sehr schwer ohne Verletzung des Gewebes zu erhalten sind. Die Einsatzmöglichkeit von Verfahren, bei denen Messelektroden außerhalb des Schädels an der Kopfoberfläche bleiben können (Elektroenzephalographie, EEG), sind auf eine räumlich vergleichsweise schlecht aufgelöste Messung der Aktivität größerer Neuronenpopulationen beschränkt.

Die präzisesten Aktivitätsdaten erhält man mit Elektroden, die in das Hirngewebe endringen und somit in unmittelbare Nähe der abzuleitenden Neuronen gelangen. Allerdings wird durch das Eindringen der Elektroden unweigerlich Nervengewebe verletzt und werden Nervenverbindungen zerstört. Aufgrund der hochgradigen Vernetzung der Neuronen ist damit immer ein Risiko verbunden, dass wichtige neuronale Funktionen eingeschränkt werden. Für wissenschaftliches Arbeiten an Tieren mag man diese Verletzung noch hinnehmen. Spätestens beim therapeutischen Einsatz am menschlichen Gehirn steht man aber vor dem Dilemma, zwischen den durch die Elektroden zugefügten Verletzungen und dem Nutzen der Therapie abwägen zu müssen.

Ein Teilausweg ist, statt in das Nervengewebe eingesetzter Elektroden solche Elektroden zu verwenden, die zwar eine Öffnung des Schädels erfordert, dort aber lediglich auf die Oberfläche des Gehirns aufgesetzt werden. Dabei bleibt das Hirngewebe selbst unverletzt. Auf diese Weise werden die gemessenen Signale von Neuronengruppen in unmittelbarer Nähe der äußeren Gehirnoberfläche dominiert.

Der Nachteil dieses Teilauswegs ist schon genannt: Bereiche des Gehirns, die nicht direkt an den äußeren Oberflächen liegen, bleiben nur teilweise erreichbar. Damit sind die Einsatzmöglichkeiten zwar gegenüber den herkömmlichen Verfahren, die außerhalb des Schädels messen, vergrößert, bleiben aber dennoch beschränkt.

Neben der Ableitung von Neuronenaktivität, bei der die aus der Aktivität der Neuronen resultierende elektrische Spannung gemessen wird, kann auch der umgekehrte Weg von Interesse sein. Dabei werden Neuronen mit elektrischen Impulsen stimuliert. Das oben geschilderte Grundproblem besteht hier in gleicher Weise: Die Stimulationselektrode muss ebenso wie die Ableitelektrode in die unmittelbare Nähe der betreffenden Neuronen gelangen, um möglichst spezifisch zu stimulieren.

Ein wichtiges Anwendungsgebiet, bei dem die genaue Kenntnis der neuronalen Aktivität eine Rolle spielt, ist die erst in jüngster Zeit entstehende motorische Neuroprothetik. Sie zielt auf Patienten mit Lähmungen, bei denen eine organische Heilung nicht mehr möglich ist, obwohl der Kortex oder zumindest der Motorkortex als derjenige Bereich, der für die Kontrolle willkürlicher Bewegungen wesentlich ist, wenigstens noch teilweise intakt ist, die Nervenverbindungen zur Muskulatur aber unterbrochen sind. Die wichtigste und insgesamt sehr häufige Ursache für solche Lähmungen sind ischämische Hirninfarkte bzw. intrazerebrale Blutungen ("Schlaganfall").

Ein besonders schwerwiegender Fall sind die so genannten locked-in-Patienten, denen durch eine vollständige Lähmung der Skelettmuskulatur jegliche willkürliche Bewegungsmöglichkeit genommen ist. Solche Patienten sind bei vollem Bewusstsein ihrer Lage zur reinen Passivität verurteilt. Genauso kann man aber auch an Patienten denken, die eine Extremität verloren haben oder die querschnittsgelähmt sind. Die Lähmung oder Behinderung hindert diese Patienten, intendierte Bewegungen auszuführen.

Die motorische Neuroprothetik hat zum Ziel, die Bewegungsfähigkeit durch willentliche Ansteuerung einer Prothese mittels eigener Hirnsignale herzustellen, wiederherzustellen oder zu verbessern. Dazu ist eine genaue Kenntnis der neuronalen Aktivität, hier im Motorkortex, eine Grundvoraussetzung. Ebenso mag man aber auch an den umgekehrten Fall denken, bei dem sensorische Signale der gelähmten Körperteile, wie etwa eine Berührung, das Gehirn nicht mehr erreichen. Dann kann eine Stimulation von Neuronen des zuständigen Hirnareals - beispielsweise des somatosensorischen Kortex - die unterbrochene körpereigene Signalübertragung ersetzen.

In allen beschriebenen Fällen erschweren die herkömmlichen Methoden entweder die Präzision der Vorhersage, oder sie verletzen intaktes Hirngewebe in einem Maße, dass der Einsatz entweder, im Tierversuch, den Erkenntnisgewinn beeinträchtigt oder, beim Menschen, die Therapie einschränkt oder gar verhindert wird.

Aus der US 2004/0082984 A1 ist eine Sonde bekannt, welche zum Zwecke der Kältetherapie in ein Gehirn eingesetzt werden kann. Die Sonde weist eine im Wesentlichen zylindrische Form auf. Auf der Mantelfläche der zylinderförmigen Sonde sind Elektroden angeordnet. Die Elektroden sind jeweils mit einer außerhalb der Sonde angeordneten Steuereinheit gekoppelt. Die Elektroden können als Stimulationselektroden oder als Ableitelektroden verwendet werden.

Aus der WO 00/09008 ist ein Verfahren zur Herstellung einer Sonde bekannt, bei welchem die Geometrie des Gehirns auf der Basis von MRi Bildern bestimmt wird.

Im Inneren der zylinderförmigen Sonde ist ein Rohr angeordnet, welches ebenfalls eine im Wesentlichen zylindrische Form aufweist. Der Sonde wird über das Rohr ein Kältemittel zugeführt. Über den Hohlraum, welcher zwischen dem Rohr und der Sonde gebildet wird, wird das Kältemittel wieder nach außen geführt.

Daher ist es Aufgabe der Erfindung, bei Vermeidung von Himgewebeverletzung eine präzise Auswertung von Signalen einer großen Zahl von Neuronen zu ermöglichen.

Diese Aufgabe wird durch eine Sonde gemäß Anspruch 1 bzw. einem Verfahren gemäß Anspruch 14 gelöst. Ein erfindungsgemäßes Herstellungsverfahren für eine Sonde ist in Anspruch 10 angegeben. Dabei geht die erfinderische Lösung von dem Prinzip aus, die Morphologie des Gehirns auszunutzen und die Messungs-/Stimulationselektrode anzupassen, statt wie sonst üblich in Umkehrung per invasivem Eingriff durch die Messung/Stimulation dem Gewebe die Gestalt der Elektrode und eine resultierende Verletzung aufzuzwingen.

Erfindungsgemäß bereitgestellt wird also eine Sonde zur Datenübertragung zwischen einem Gehirn (2) und einer Datenverarbeitungsvorrichtung (5) gemäβ Anspruch 1.

Die Elektroden sind also flächig bzw. punktförmig ausgestaltet. Auf diese Weise kann die Sonde Neuronen beider Seitenwände erreichen und sie je nach Ansteuerung stimulieren oder ableiten. Damit ermöglicht die Sonde, eine besonders große Zahl von Neuronen zu erreichen, die zu beiden Seiten des Sulcus liegen. Das wäre mit einer invasiven Elektrode nur mit hohem Aufwand und entsprechender Gewebeverletzung, mit einer Oberflächenelektrode gar nicht möglich.

Die Lösung hat den Vorteil, dass die Elektroden und die Sonde das Gehirn unverletzt lassen können. Damit sind aber auch diverse Risiken bei einer Operation zum Einsatz der Elektroden vermindert. Gleichzeitig ist die Langzeitverträglichkeit gut - ein ungewolltes Verschieben der Elektroden ist kaum möglich, weil der Träger in die Form des Sulcus gebracht und somit an individuelle Hirnfurchen bzw. -windungen angepasst ist, sich anschmiegen kann und so an Ort und Stelle gehalten wird. Somit bleiben die Signale stabil, weil die benachbarten Neuronen stets dieselben sind, und außerdem sind keinerlei scharfe Kanten oder Spitzen vorhanden, die bei doch einmal auftretendem Verschieben des Trägers - etwa durch besonders ruckartige Bewegungen bei einem Unfall - Verletzungen hervorrufen könnten. Schon bei normalen Bewegungen lassen sich solche Verletzungen bei herkömmlichen intrakortikal implantierten Elektroden nicht wirksam verhindern. Schließlich werden durch diese Lösung dennoch Areale des Gehirns und insbesondere des Kortex zugänglich gemacht, die für die unten beschriebenen Anwendungsfälle interessant oder gar notwendig sind.

Vorteilhafterweise gehören die Neuronen der ersten und der zweiten Seitenwand unterschiedlichen funktionellen Arealen des Gehirns zu. In diesem Fall trennt der Sulcus zwei funktionelle Areale. Damit kann von jeder Seite der Sonde ein eigenes funktionelles Areal angesprochen werden.

In vorteilhafter Weiterbildung gehören die Neuronen der ersten Seitenwand dem Motorkortex und die Neuronen der zweiten Seitenwand dem somatosensorischen Kortex zu. Der Motorkortex ist ein typischer ausgabeorientiertes Areal, der somatosensorische Kortex umgekehrt ist ein eingabeorientiertes Areal. Ein und derselbe Träger kann in dieser Weiterbildung sowohl der motorischen Ableitung wie der somatosensorischen Stimulation dienen.

Bevorzugt weist die erste Gruppe Ableitelektroden und die zweite Gruppe Stimulationselektroden auf. Diese Zuordnung ist besonders vorteilhaft, wenn Ableitelektroden einem ausgabeorientierten Areal und Stimulationselektroden einem eingabeorientierten Areal zugeordnet sind. Dennoch muss die Aufteilung keine ausschließliche sein, weil auch die Stimulation eines ausgabeorientierten Areals oder die Ableitung von einem ausgabeorientieren Areal sinnvoll sein kann.

Bevorzugt weist der Träger Polyimid oder Silikon auf. Diese Materialien haben sich als gut verarbeitbar, biokompatibel oder biologisch verträglich und langzeitstabil erwiesen.

Bevorzugt sind die Vielzahl von Elektroden mit einer Dichte zwischen einem und 1000 Elektrodenkontakten pro cm² auf dem Träger aufgebracht. Die Einschränkung der oberen Grenze von 1000 Elektrodenkontakten pro cm² kann selbstverständlich angehoben werden, sofern die entsprechende Technik gewählt wird und die Anwendung es erfordert. Je nach der interessierenden Neuronenpopulation kann somit die Balance zwischen räumlicher Auflösung auf der einen Seite und Rechenkomplexität sowie Empfindlichkeit der Elektroden auf der anderen Seite gewählt werden.

Bevorzugt weisen die Elektroden Gold, Platin, eine metallische Legierung, leitenden Kunststoff oder Halbleitermaterialien auf. Insbesondere die Metalle eignen sich gut wegen guter Mess-/Stimulationsergebnisse und ihrer Langzeitstabilität und Verträglichkeit. Leitende Kunststoffe oder Halbleiter lassen sich besonders gut mit dem flexiblen Träger verarbeiten.

Die Einrichtung zur Datenübertragung, welche eine erfindungsgemäße Sonde aufweist, ist vorteilhafterweise dafür ausgebildet, einen ersten Teil der Elektroden mittels Auslesen der Eingangssignale als Ableitelektroden und einen zweiten Teil der Elektroden mittels Zuführen der Ausgangssignale als Stimulationselektroden anzusteuern, so dass ein bidirektionaler Datenaustausch ermöglicht ist. Damit wird die Möglichkeit der Sonde ausgenutzt, die Elektroden in einer der beiden Richtungen anzusteuern. Jede Elektrode kann sowohl neuronale Aktivität messen als auch elektrische Impulse abgegeben. Bei dieser Ansteuerung kann den Elektroden je nach Bedarf eine dieser Rollen zugewiesen werden. Die Einrichtung ermöglicht somit nicht nur einen Übertragungsweg, sondern beide. Herkömmlich müsste dazu eine so große Zahl invasiver Elektroden eingesetzt werden, dass der Gesamtnutzen bezweifelt werden darf. Eine einzige Oberflächenelektrode, die verschiedene funktionale Areale zur Stimulation und Ableitung erreicht, ist ebenfalls schwer vorzustellen; sie müsste zumindest zweiteilig sein, will man übermäßige Größe vermeiden. Das erschwert natürlich wiederum die Operation, Positionierung, und Langzeitstabilität.

Bevorzugt ist die Auswerteeinrichtung zusätzlich als Effektorsteuerung eines anschließbaren Effektors ausgebildet und berechnet unter Einbeziehung der Eingangssignale Effektorsteuersignale für den Effektor und/oder unter Einbeziehung von Effektorzustandsignalen des Effektors die Stimulationssignale. Die elektromagnetischen Signale von den Neuronen werden also nicht lediglich aufgezeichnet, sondern können unmittelbar für die Steuerung eines Effektors verwendet werden. Umgekehrt kann der Effektor auf diese Weise kontrolliert die neuronale Aktivität beeinflussen.

Bevorzugter ist die Einrichtung derart ausgestaltet, dass
- der Effektor eine Prothese ist;
- die Eingangssignale diejenigen von Neuronen im Motorkortex und die Stimulationssignale für Neuronen im somatosensorischen Kortex sind;
- die Effektorsteuersignale Bewegungsparameter der Prothese beeinflussen und
- die Effektorzustandssignale Stellungs-, Bewegungs- und/oder Zustands-parameter weiterer Sensoren wie Druck-, Berührungs-, Abstands- oder Temperatursensoren sind,
so dass das Gehirn die Bewegung der Prothese steuern kann und unmittelbare somatosensorische Rückmeldung über die Bewegung sowie die Umgebung der Prothese erhält.

Auf diese Weise wird dem Patienten nicht nur die willentliche Steuerung der Prothese ermöglicht. Er erhält darüber hinaus auch sensorische Rückmeldung, also ein Gefühl seines ersetzten Körperteils zurück.

Alternativ ist die Einrichtung derart ausgestaltet, dass
- der Effektor ein Körperteil des Lebewesens ist;
- die Eingangssignale diejenigen von Neuronen im Motorkortex und die Stimulationssignale für Neuronen im somatosensorischen Kortex sind;
- die Effektorsteuersignale Motorneuronen oder Muskelfasern des Körperteils beeinflussen und
- die Effektorzustandssignale Signale von Rezeptoren oder Rezeptorneuronen des Körperteils und/oder Stellungs-, Bewegungs- und/oder Zustandsparameter weiterer Sensoren wie Druck-, Berührungs-, Abstands- oder Temperatursensoren sind,
so dass das Gehirn die Bewegung des Körperteils steuern kann und unmittelbare somatosensorische Rückmeldung über die Bewegung sowie die Umgebung des Körperteils erhält.

Auf diese Weise wird die Kontrolle über das Körperteil sowohl hinsichtlich dessen Bewegung als auch hinsichtlich dessen Gefühls zurückgegeben.

Alternativ ist die Einrichtung derart ausgestaltet, dass
- der Effektor ein Rechner insbesondere mit einer Anzeige ist;
- die Eingangssignale diejenigen von Neuronen im Motorkortex und die Stimulationssignale für Neuronen im somatosensorischen Kortex sind;
- die Effektorsteuersignale virtuelle, insbesondere auf der Anzeige dargestellte Bewegungen oder Funktionen in dem Rechner beeinflussen und
- die Auswerteeinrichtung unter Einbeziehung der virtuellen Bewegung oder der Funktion die Effektorzustandssignale errechnet.

Der virtuelle Effektor hat den großen Vorteil, in seiner Funktionalität äußerst variabel, dabei kostengünstig und praktisch unbegrenzt verfügbar und frei konfigurierbar sowie von mechanischen Problemen jeglicher Art befreit zu sein. Selbst im Falle von geringerer Qualität der abgeleiteten Signale kann hier noch eine sehr nützliche Funktion ausgelöst und deren Feedback vermittelt werden.

Vorteilhafterweise ist ein Verstärker vorgesehen, der für die Verstärkung und Filterung von den Eingangssignalen zu vorverarbeiteten Eingangssignalen und/oder von den Ausgangssignalen zu den Stimulationssignalen ausgebildet ist. Die neuronalen Signale, welche die Elektroden ableiten, bedürfen oftmals einer Aufbereitung, ehe mit ihrer Auswertung begonnen werden kann. Umgekehrt müssen natürlich auch die Stimulationssignale von für das Neuron verarbeitbarer Beschaffenheit sein.

Bei dem erfindungsgemäßen Herstellungsverfahren einer Sonde wird vorteilhafterweise die Geometrie des Sulcus durch Auswertung nichtinvasiver Bildgebungsverfahren erfasst. Dazu zählen Computertomographie (CT) und Magnetresonanztomographie (MRT) sowie die funktionelle Magnetresonanztomographie (fMRT) und ähnliche aus der Forschung bekannte Verfahren. Der Vorteil ist, dass einerseits dem Patienten vor dem späteren eigentlichen Einsetzen der Sonde kein weiterer Eingriff zugemutet werden muss und dass andererseits vermieden wird, dass sich der Eingriff verlängert oder die Qualität der Sonde verringert, weil die Formgebung unter Zeitdruck während dieses Einsetzens vorgenommen werden muss. Im Übrigen wird natürlich eine besonders genau angepasste Form durch die nach dem Bildgebungsverfahren gut bekannte Geometrie ermöglicht.

Bevorzugt sind die Elektroden auf dem Träger in einer an die Morphologie des Innenraums angepassten Weise angeordnet. Damit passt sich nicht nur der Träger selbst, sondern auch die eigentlichen Informationsüberträger an die Erfordernisse im Gehirn an. Das kann sowohl die Geometrie als solche wie auch andere morphologische Erfordernisse betreffen, etwa Neuronendichte oder -größe, deren Grad an Vernetzung, Stärke ihrer elektromagnetischen Felder oder ähnlichem betreffen, die dann in der Elektrodenanordnung, -größe, -empfindlichkeit etc. nachgebildet werden kann.

Das Herstellungsverfahren zeigt in den sich anschließenden Unteransprüchen beispielhaft, aber nicht abschließend noch weitere Merkmale und Vorteile, die denjenigen der oben beschriebenen Sonde selbst ähnlich sind.
Auch das erfinderische Verfahren zur Datenübertragung, welches eine in einen Sulcus eingesetzte erfindungsgemäße Sonde aufweist, zeigt ähnliche und weitere Merkmale und Vorteile, wie sie beispielhaft, aber nicht abschließend, in den sich anschließenden Unteransprüchen beschrieben sind.

Die Erfindung wird nachstehend auch hinsichtlich weiterer Merkmale und Vorteile unter Bezugnahme auf die beigefügte Zeichnung detailliert beschrieben. Die Zeichnung zeigt in:
- Fig. 1a: eine in das Gehirn eingesetzte Ausführungsform der Erfindung in der Draufsicht;
- Fig. 1b: eine Querschnittsdarstellung der Ausführungsform gemäß Fig. 1a entlang der Strichlinie in Fig. 1a;
- Fig. 1c: eine Querschnittsdarstellung einer alternativen Ausführungsform der Erfindung;
- Fig. 2a: eine Seitenansicht der vorderen Trägeroberfläche und Elektroden der Ausführungsform gemäß Fig. 1;
- Fig. 2b: eine Seitenansicht der rückwärtigen Trägeroberfläche und Elektroden der Ausführungsform gemäß Fig. 1;
- Fig. 3: eine Schnittansicht des Trägers gemäß Fig. 2;
- Fig. 4: eine perspektivische Sicht des Trägers;
- Fig. 5: eine Übersichtsdarstellung einer eingesetzten Ausführungsform der Erfindung und vorteilhafte Peripherie;
- Fig. 6: eine Darstellung einer Armprothese als Beispiel eines Effektors, der von einer Ausführungsform der Erfindung angesteuert werden kann;
- Fig. 7: eine schematische Darstellung der Signalübertragungsschnittstelle für eine Ausführungsform der Erfindung;
- Fig. 8: eine beispielhafte Schemadarstellung der Umrechnung von neuronalen Signalen Daten in Steuerungssignale für einen Effektor;
- Fig. 9: eine beispielhafte Schemadarstellung der Umrechnung von Feedbackdaten eines Effektors in Stimulationssignale für die Elektroden; und
- Fig. 10: ein Diagramm des erfindungsgemäßen Herstellungsverfahrens.

Die Großhirnrinde (Cortex cerebri) des menschlichen Gehirns hat eine stark gewundene Form, bei der Sulci (Furchen) die einzelnen Gyri (Hirnwindungen) voneinander trennen. Es ist wichtig zu betonen, dass zwar die medizinischen Anwendungen zunächst auf den Menschen fokussiert sein werden. Dennoch ist die Erfindung nicht auf das menschliche Gehirn beschränkt, sondern überall da anwendbar, wo ein Gehirn auch eines Tieres gyrencephale Gestalt hat (d.h. über Furchen und Hirnwindungen verfügt). Dies muss nicht (ausschließlich) zu therapeutischen, sondern kann (auch) zu neurowissenschaftlichen Zwecken erfolgen.

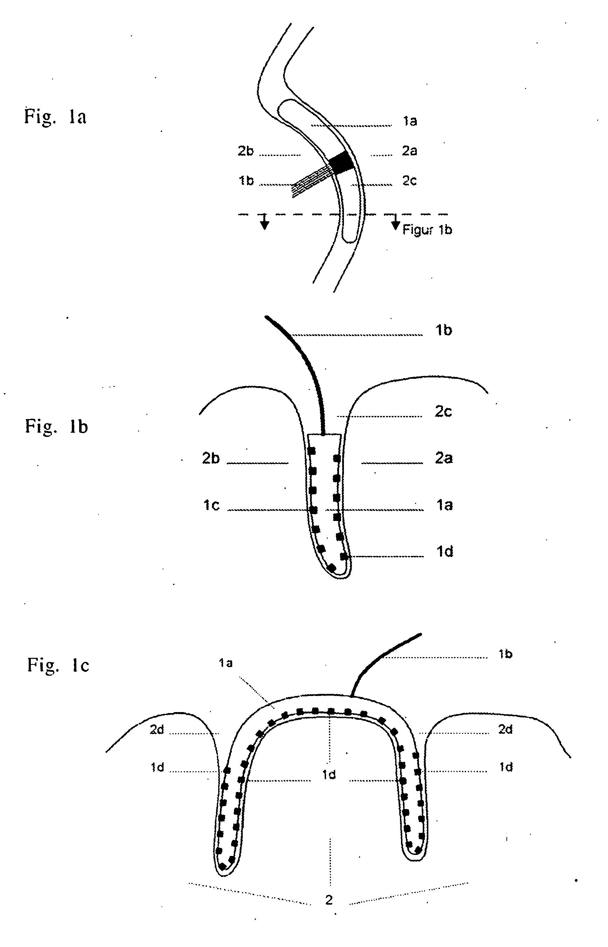

Fig. 1a bis 1c zeigen eine eingesetzte Ausführungsform der Erfindung und ihre Anordnung bezüglich des Gehirns in Draufsicht bzw. im Querschnitt. Eine Multielektrode 1 mit einem Träger 1a aus flexiblem oder elastischem Material ist in einen Sulcus 2c eingebettet, der von zwei seitlichen Oberflächen der benachbarten Hirnwindungen 2a, 2b begrenzt ist. Die Multielektrode 1 ist somit eine corticomorphe Elektrode, die an die Form der Gehirnoberfläche angepasst werden bzw. sich dieser selbst anpassen kann.

Der Träger 1a ist daher in seiner Form genau an die Oberflächenform der Hirnwindungen angepasst, so dass er sich gut anschmiegen kann. Aus Stabilitätsgründen kann es sich empfehlen, den Träger 1a bis zum Boden des Sulcus 2c einzusetzen. Das ist aber nicht zwingend, wenn höher gelegene seitliche Oberflächen berührt werden sollen, für welche die Höhe des Trägers 1a nicht hinreicht. Als Material des Trägers 1a bieten sich Polyimid oder Silikon wegen ihrer Verträglichkeit, leichten Verarbeitbarkeit und Unempfindlichkeit an. Genauso ist aber auch jedes andere Material geeignet, das die erforderliche Flexibilität und Biokompatibilität besitzt. Das Material sollte darüber hinaus natürlich nicht leitend sein. Es sollte in einfacher Weise ermöglichen, dem Träger seine individuelle Form zu geben, also beispielsweise leicht zurechtzuschneiden zu sein. Schließlich muss der Träger elastisch und dünn genüg sein, meist bei einer Dicke <<1cm. Um kein Gewebe zu verletzen, hat der Träger abgerundete Kanten.

Auf dem Träger befinden sich eine Reihe von Elektroden 1c, 1d, welche jeweils einzeln mit einem Kabel 1b verbunden sind, über das Signale nach außen oder von außen geleitet werden können. Die genaue Struktur der Elektroden 1c, 1d auf dem Träger 1a und der Leitungsverbindungen wird unten noch genauer dargestellt. Wegen der sich anschmiegenden Gestalt des Trägers 1a kommen die auf seiner Oberfläche angeordneten Elektroden 1c, 1d unmittelbar in Kontakt mit der Gehirnoberfläche 2a, 2b und können so gut mit Neuronen des angrenzenden Gehirngewebes 2a, 2b in elektromagnetische Wechselwirkung treten. Üblicherweise wird das Gehirngewebe 2a, 2b in der einen Signalrichtung durch elektrische Ströme stimuliert bzw. dessen Aktivität in der anderen Richtung über die elektrische Spannung gemessen. Das bedeutet aber nicht, dass die Erfindung darauf beschränkt ist. So umfasst die Erfindung auch, über die Spannung zu stimulieren, Ströme zu messen oder beliebige andere elektrische oder magnetische Größen zu messen bzw. zu beeinflussen. Wichtig ist, dass jede Elektrode 1c, 1d je nach Ansteuerung Aktivität der Neuronen messen oder diese mittels elektromagnetischer Impulse stimulieren kann.

Eine besondere Ausführungsform der Sonde ist für den zentralen Sulcus 2c zwischen dem primären somatosensorischen Kortex 2a und dem primären motorischen Kortex 2b bestimmt. In diesem Falle sind die Rollen für die Elektroden 1c, 1d so verteilt, dass diejenigen Elektroden 1c in Kontakt mit der Oberfläche des primären motorischen Kortex 2b als Mess- oder Ableitungselektroden 1c angesteuert werden, während diejenigen Elektroden 1d in Kontakt mit der Oberfläche des somatosensorischen Kortex 2a als Stimulationselektroden 1d angesteuert werden. Diese Zuordnung passt zu der Aufgabe des somatosensorischen Kortex 2a, der im intakten Gehirn vornehmlich eingehende Informationen verarbeitet, während andererseits der primäre motorische Kortex 2b für die Bewegungsplanung und Durchführung zuständig ist und demnach bei funktioneller Betrachtung Ausgangssignale an nachgeordnete Gehirn- und Rückenmarksteile überträgt. Es ist durchaus vorstellbar, diese strenge Einteilung der Elektroden 1c, 1d anders vorzunehmen und etwa auch im primären motorischen Kortex 2b zu stimulieren. Vorstellbar wäre, z. B. funktionelle neuronale Aktivitätsmuster im primären motorischen Kortex 2b durch eine Stimulation auszulösen, zu testen oder zu unterstützen bzw. zu verstärken. Dies ist also eine Frage der Ansteuerung und der Anwendung, die Erfindung ist auf eine strenge Einteilung der beschriebenen Art nicht beschränkt.

Obwohl ein Einsatz an dem somatosensorischen Kortex 2a und dem primären motorischen Kortex 2b hier als wichtiges Anwendungsbeispiel besonders gründlich beschrieben wird, ist die Erfindung hierauf nicht beschränkt. Prinzipiell ist die erfindungsgemäße Sonde für jeden Sulcus geeignet, und bogenförmige Elektroden können auch einer Hirnwindung angepasst werden und so von einem Sulcus in einen benachbarten Sulcus reichen. Dieser Fall ist schematisch in einem der Fig. 1b, wo der Träger in einen einzigen Sulcus eingesetzt dargestellt ist, analogen Querschnitt in der Fig. 1c gezeigt.

Eine Operation, mit welcher der Träger 1a eingesetzt wird, bedarf einer spezifischen prächirurgischen Diagnostik und OP-Planung. Einer der wichtigsten Aspekte ist, das exakte Zielgebiet für eine Implantation zu bestimmen, das aufgrund der starken inter-individuellen neuroanatomischen Variabilität des menschlichen Gehirns nicht a priori festgelegt werden kann. Nur in Ausnahmefällen wäre ein Einsetzen an einer Stelle erwünscht, die nicht zuvor individuell bestimmt wurde. Zwar kennt man allgemeine Kartierungen des Gehirns und weiß daher, wo bestimmte funktionelle Areale zu finden sind. Im spezielleren Beispiel des Motor-und des somatosensorischen Kortex ist sogar die Anatomie des Menschen örtlich nachgebildet und einzelne Körperteile räumlich distinkten Gebieten des Kortex zugeordnet. Für den einzelnen Patienten ist dieses Vorwissen zumeist dennoch zu grob.

Eine exakte Lokalisation der Zielgebiete für einen individuellen Patienten wird daher präoperativ durch Anwendung von fMRT vorgenommen. Hierbei wird ortspezifische Aktivierung des Gehirns gemessen, während der Patient die Steuerung des Effektors versucht, sich vorstellt oder beobachtet, und so der Implantationsort mit hoher räumlicher Genauigkeit bestimmt werden kann. Ergänzend kann, zur Verbesserung der Lokalisation, eine EEG-Messung mit anschließender Quellenrekonstruktion während derselben motorischen Paradigmen (Versuchen, Vorstellen oder Beobachten der Effektorsteuerung) durchgeführt werden.

Anhand eines anatomischen MRT-Bildsatzes wird die dreidimensionale Geometrie des Sulcus 2c vermessen und der Träger 1a mit dessen Hilfe in eine Form gebracht, die genau in den Spaltraum oder Innenraum des Sulcus passt, ihn so nach der Implantation stabil gegen Kopfbewegungen macht und in guten Kontakt mit den Seitenwänden 2a, 2b bringt. Eine gewisse Fehlertoleranz ist durch die Flexibilität des Gehirngewebes geweben.

Selbstverständlich steht es in der Anwendung frei, den Träger 1a ohne diese aufwändigen Vorverfahren einzusetzen. Das dürfte selbst im Tierversuch, erst recht natürlich für Patienten selten optimal sein. Die Erfindung schließt es aber keineswegs aus. Lediglich eine gewisse Anpassung der Form des Trägers 1a ist unabdingbar, die aber nicht notwendig auf einer Vermessung des Individuums basieren muss, in das er eingesetzt werden soll, sondern z. B. auch auf Erwartungen, theoretischen Vorhersagen oder Erfahrungsdaten basieren kann..

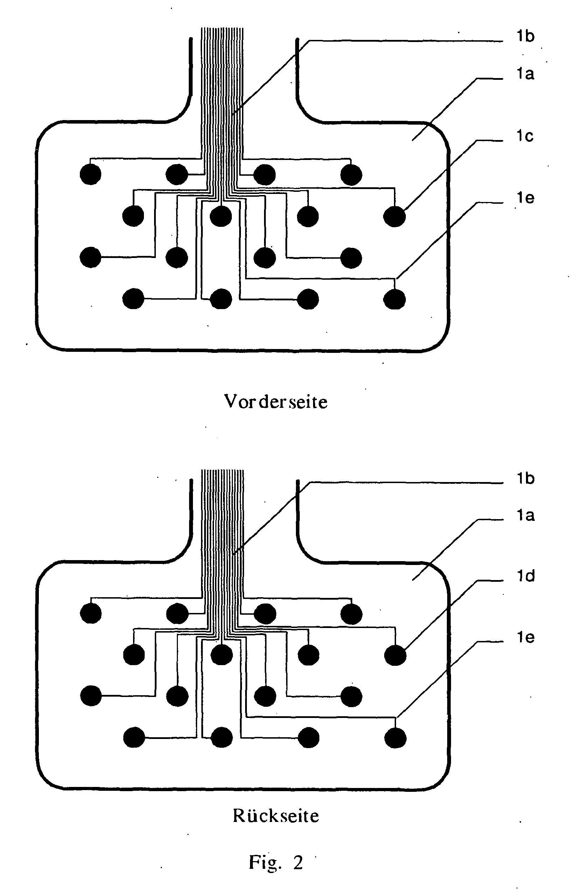

Unter Bezugnahme auf die Figuren 2a und 2b soll nun die Gestalt des Trägers 1a und Anordnung sowie Verbindungen der Elektroden 1c, 1d erläutert werden. Dabei zeigt Fig. 2a die Vorder- und Fig. 2b die Rückseite des Trägers 1a. Die Figuren beziehen sich auf das Ausführungsbeispiel, bei dem eine Oberfläche des Trägers 1a mit einem zu stimulieienden und die andere mit einem zu messenden Hirnareal in Verbindung steht. Darauf ist, bei anderer Anordnung der Elektroden 1c, 1d und ihrer Verbindungen, die Erfindung jedoch nicht beschränkt.

Der Träger 1a ist in grober Näherung rechteckig dargestellt. Manchmal kann dies in der Anwendung genügen. Der Träger kann beispielsweise als einfache oder als Doppelfolie aufgelegt sein. In einer an den Sulcus 2c angepassten Ausführungsform ist das Trägermaterial aber so modelliert, dass es die Gestalt der Begrenzungen des Sulcus 2c annimmt. Dabei sind die Dimensionen zu beachten; der Sulcus 2c lässt nur eine sehr geringe Dicke des Trägers 1b zu.

Der Träger 1a besteht gewöhnlich aus einem flexiblen Material. Wenn der Träger 1a entsprechend vormodelliert ist, kommen auch andere Materialien in Betracht, sofern sie das Einsetzen in den Sulcus 2c nicht zu sehr erschweren. In jedem Fall sollte das Material biokompatibel sein, also das Hirngewebe möglichst auch noch bei Langzeiteinsatz nicht beeinträchtigen. Polyimid oder Silikon ist hierfür ein geeignetes Trägermaterial, ohne dass die Erfindung auf dieses Material beschränkt wäre.

Die Elektroden 1c, 1d sind als Kontaktpunkte bzw. Kontaktflächen matrixförmig angeordnet. Die Oberfläche des Trägers 1a mit den Kontaktpunkten bzw. Kontaktflächen ist im Wesentlichen eben ausgestaltet. Leiterbahnen 1e im Inneren des Trägers 1b verbinden jede Elektrode 1c, 1d einzeln und ohne Überlappung ihrer jeweiligen Leiterbahnen 1e mit dem Kabel 1b für den Signalaustausch. Das Kabel 1b ist mindestens zweiteilig ausgelegt, wobei eine Messleitung 1b1 Signale der Messelektroden 1c nach außen und eine Stimulationsleitung 1b2 Signale für die Stimulationselektroden überträgt. Es ist auch ein einteiliges Kabel 1b vorstellbar, das jeweils in verschiedenen Zeitintervallen für die Übertragung von Messdaten nach außen und von Stimulationsdaten nach innen verwendet wird. Die Herstellungsmöglichkeiten solcher Leiterbahnen 1e und Möglichkeiten ihrer Anordnung kennt der Fachmann.

Auch Elektroden können aus verschiedenem Material hergestellt werden, insbesondere Gold, Platin, einer metallischen Legierung oder auch aus leitenden Kunststoffen sowie Halbleitermaterialien. Der Träger 1a kann eine Größe von weniger als einem bis zu mehr als zehn Zentimetern annehmen. Die Elektrodenkontakte sind mit einer typischen Dichte von 1 bis zu mehr als 10.000 Elektrodenkontakten pro cm² ausgelegt. Eine höhere Dichte an Elektrodenkontakten verbessert die Signalauflösung, erhöht aber natürlich den Aufwand nicht nur der Herstellung der Elektroden 1c, 1d, sondern auch den der Verstärkung und den Rechenaufwand der Ansteuerung.

Selbstverständlich kann die Anordnung je nach Bedarf von der hier dargestellten mit gegeneinander versetzten Reihen abweichen. Hier ist praktisch jede Anordnung einer Punkteschar auf einer (Ober)fläche möglich.
Wie z.B. Fig. 1b und 1c illustrieren, müssen die beiden Gruppen von Elektroden z.B. nicht symmetrisch zu einer Schnittebene durch den Träger 1a angeordnet sein. Vielmehr können die Elektroden der einen Oberfläche versetzt sein gegenüber den Elektroden der anderen Oberfläche oder beliebigen anderen Anordnunggen entsprechend den Ergebnissen der fMRT-Auswertung. Auch kann eine Oberfläche des Trägers 1c vollkommen oder abschnittsweise frei von Elektroden sein, wie z.B. Fig. 1c illustriert.

Es ist besonders vorteilhaft, dass dieser Träger das Hirngewebe im Gegensatz zu eindringenden Elektroden nicht verletzt. Damit wird auch eine bessere Langzeitstabilität der Signalerfassung erreicht, weil in das Hirngewebe eindringende Elektroden zu einer lokalen Gewebezerstörung und damit dem Erlöschen lokaler neuronaler Aktivität führen können. Je nach Anwendung kann der Träger 1a mit den Elektroden 1c, 1d auch sehr klein sein (<<1cm). In diesem Fall ist die Operation, mit der der Träger 1a dem Patienten eingesetzt wird, mit sehr geringem Aufwand und sehr geringer Beeinträchtigung des Patienten möglich.

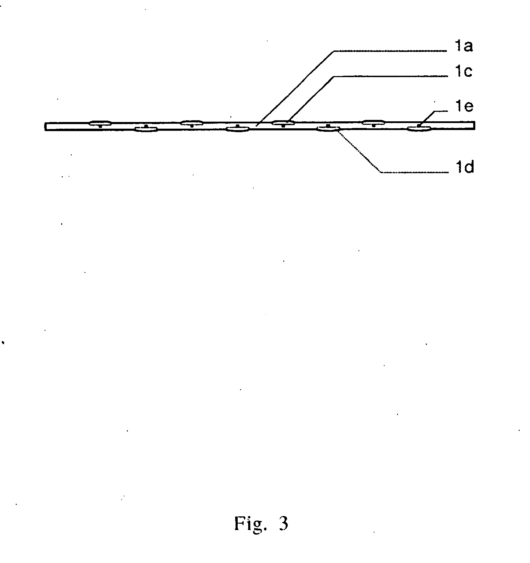

Fig. 3 zeigt den Träger in einer Schnittansicht, also im Profil. Wie zu erkennen ist, weist der Träger zwei flache Oberflächen auf.

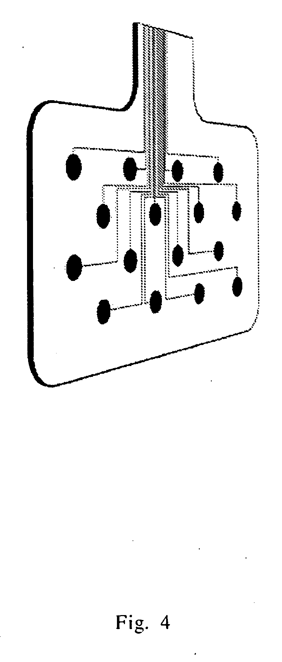

Fig. 4 illustriert den Träger 1a in einer perspektivischen Sicht.
Die Sonden werden durch den im Folgenden beschriebenen technischen Prozess hergestellt, d.h. individuell auf das Gehirn des Patienten abgestimmt.

Durch Anwendung struktureller bildgebender Verfahren werden Informationen über die exakte Anatomie des Cerebralen Cortex des Gehirns gewonnen. Hierzu werden vorteilhafterweise T1-gewichtete MRT-Aufnahmen verwendet, da sie ohne eine Belastung durch ionisierende Strahlung gewonnen werden können. Des Weiteren können durch funktionelle bildgebende Verfahren Informationen über die Lokalisation von Gehirnbereichen gewonnen werden, die willkürliche Bewegungen steuern. Hier sind insbesondere Verfahren der funktionellen MR-Bildgebung (fMRT) aufgrund ihrer hohen räumlichen Auflösung vorteilhaft.

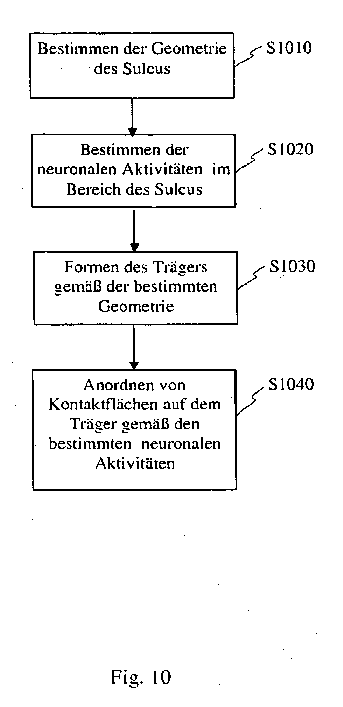

Information aus strukturellen und/oder funktionellen bildgebenden Verfahren kann verwendet werden, um folgende Eigenschaften der zu implantierenden Elektroden möglichst genau an das Gehirn des individuellen zu behandelnden Patienten anzupassen, vgl. hierzu auch Fig. 10:
- Größe der Sonde;
- Form der Sonde;
- Anordnung der einzelnen Kontaktflächen auf dem Träger 1a der Sonde;
- Zahl der insgesamt zu implantierenden Sonden;
- Positionierung der Sonde auf dem Cortex.

Basis sind ein hochaufgelöster struktureller Bilddatensatz des Gehirns, vorzugsweise mit einer Auflösung von 1mm x 1mm x 1mm oder besser. Des Weiteren werden funktionelle Bilddaten während einer Testbatterie von Bewegungsaufgaben aufgenommen, die das Repertoire an natürlichen Bewegungsaufgaben, die durch das BMI letztendlich gesteuert werden sollen, möglichst weitgehend abzudecken in der Lage ist.

### Beim Herstellungsverfahren gemäß der Erfindung werden folgende Schritte durchgeführt:

Der strukturelle Bilddatensatz wird ausgewertet, um die Geometrie des Sulcus zu bestimmen (Schritt 1010).

Die funktionellen Bilddaten werden herangezogen, um die neuronalen Aktivitäten des Sulcus zu bestimmen (Schritt 1020), insbesondere mit einigen oder allen der folgenden Schritte: Korrektur von Kopfbewegungseffekten während der Messung, Beseitigung von Artefakten, Normalisierung in ein Standardkoordinatensystem, räumliche Filterung, zeitliche Filterung, statistische Auswertung basierend auf parametrischen oder auch nicht-parametrischen Verfahren.

Aus den resultierenden Aktivierungsdaten während verschiedener Bewegungsaufgaben und - optional - unter zusätzlicher Einbeziehung der strukturellen Daten wird dann der oder die Gehirnbereiche bestimmt, die die höchste Bewegungsinformation erwarten lassen. Durch entsprechende Algorithmen wird eine optimale Implantation entworfen, die möglichst hohe Bewegungsinformation bei möglichst wenigen zu implantierenden Sonden bzw. einer möglichst geringen Gesamtfläche der zu implantieren Sonden verbindet. In diesem Schritt können dabei alle oben genannten Parameter einbezogen werden. Die Daten über Parameter der einzelnen Träger werden für die individuelle Anfertigung der zu implantierenden Träger verwendet, vgl. Schritt 1030.

Die Kontaktflächen werden derart auf dem Träger 1a positioniert, dass sie den Stellen signifikanter neuronaler Aktivität in demjenigen Bereich des Sulcus entsprechen, wo der Träger 1a entsprechend den Ergebnissen der Schritte 1010 und 1020 positioniert werden soll, vgl. Schritt 1040.

Das Herstellungsveifahren liefert also eine Sonde mit Träger 1a mit spezifischer Geometrie Form und spezifisch angeordneten Kontaktpunkten.

Wenn unter neurochirurgischen Aspekten erforderlich, werden die Daten des im vorherigen Schritt optimierten Implantation auf ein Neuronavigationsgerät übertragen und die Sondenpositionierung im Gehirn so computergestützt durchgeführt.

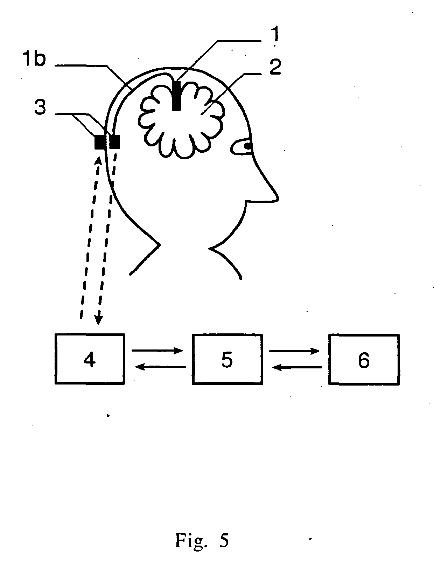

Fig. 5 zeigt eine Übersichtsdarstellung einer in das Großhirn 2 eingesetzten Ausführungsform der Erfindung und vorteilhafte Peripherie. Die Multielektrode 1 zur Ableitung der neuronalen Aktivität bzw. Stimulation ist in den Schädel des Patienten wie zuvor beschrieben in einem Sulcus 2c eingesetzt. Die Multielektrode I misst die neuronale Aktivität und leitet sie über eine noch zu beschreibende Signalschnittstelle 3 als elektromagnetische Eingangssignale an einen Verstärker 4 weiter, der vorzugsweise als Mehrkanalverstärker ausgebildet ist. In dem Verstärker 4 können zusätzlich zu einer Verstärkung Hoch-, Tief-, oder Bandpassfilter zum Einsatz kommen (beispielsweise Savitzky-Golay-, Butterworth- oder Chebychevfilter). Vorteilhaft ist eine hohe zeitliche Auflösung für Echtzeitübertragung, idealerweise liegt die Abtastrate bei mehr als 200 Hz, geringere Werte sind jedoch nicht ausgeschlossen.

Der Verstärker verstärkt und filtert die elektromagnetischen Eingangssignale und leitet die derart vorverarbeiteten Signale in Echtzeit an einen Auswertechip, einen Computer oder dergleichen System 5 zur Signalverarbeitung weiter. Bei einer Ausführungsform der Erfindung ist damit das Ziel bereits erreicht: die neuronale Aktivität wurde gemessen und kann in dem System 5 wie gewünscht ausgewertet werden.

Bei einer anderen Ausführungsform der Erfindung werden in dem System 5 Stimulationssignale erzeugt, die über den Verstärker 4 und die Signalschnittstelle 3 der Multielektrode 1 zugeführt werden, wo Einzelelektroden 1d entsprechende Stimulationsimpulse abgeben.

Wiederum in einer anderen Ausführungsform überträgt das System 5 Effektorsteuersignale an einen Effektor 6. Umgekehrt kann der Effektor 6 Effektorzustandssignale an das System 5 zurückgeben.

Es ist wichtig zu betonen, dass weitere Kombinationen der angegebenen Komponenten möglich sind. So kann die Verbindung zur Effektorsteuerung bidirektional angelegt sein, der Effektor kann aber auch unidirektional ausschließlich zu Aktionen veranlasst werden oder einseitig (als reiner Sensor) ausschließlich Zustandssignale übertragen. Ebenso kann auch die Verbindung zwischen dem System 5 und der Multielektrode 1 je nach Anwendung bidirektional oder in einer der beiden Richtungen einseitig sein. Bevorzugt ist jedoch die bidirektionale Verbindung, da so die erfinderische Anordnung der Multielektrode 1 innerhalb eines Sulcus 2c am besten ausgenutzt werden kann.

Stellvertretend für die Vielzahl an Anwendungsvarianten, bei denen die Multielektrode 1 an unterschiedlichen Hirnarealen eingesetzt wird, wird im folgenden weiter ein Einsatz im zentralen Sulcus 2c zwischen dem somatosensorischen Kortex 2a und dem primären motorischen Kortex 2b beschrieben. Das ist keineswegs einschränkend zu verstehen. Die Erfindung umfasst auch die Möglichkeit, jedes beliebige andere Gehirnareal zu stimulieren und/oder abzuleiten.

Für den Effektor 6 kommen dann im Wesentlichen drei Gruppen in Betracht: ein mechanisches Gerät wie ein Roboter, Roboterarm oder eine Prothese, ein eigenes Körperteil oder ein durch virtuellen Befehl eines Rechners angesteuertes elektrisches Gerät wie ein Computer, ein Mobilfunkgerät, ein Haushaltsgerät oder dergleichen.

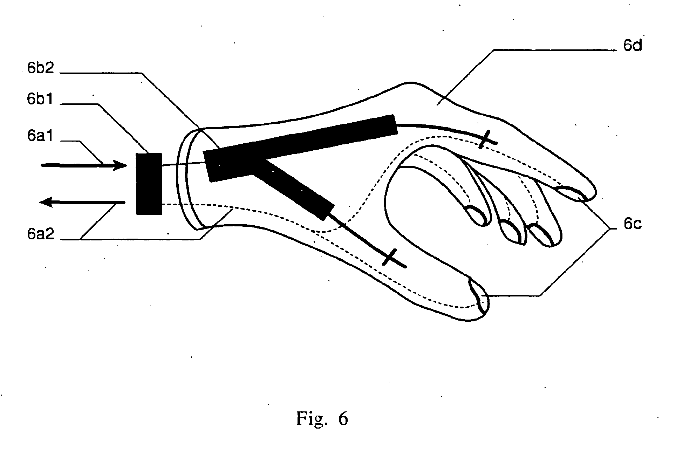

Der erste Fall einer Prothese, also einer künstlichen Nachbildung eines Körperteils, wird im Folgenden anhand einer schematisch in Fig. 6 dargestellten Handprothese näher erläutert. Selbstverständlich kann jede Art von Prothese angesteuert werden, es sind auch eher absurd anmutende Ansteuerungen wie die eines dritten Armes oder Beines denkbar.

Über eine Effektoreingangsleitung 6a1 werden die Effektorsteuerungssignale von dem System 5 an den Effektor 6 übertragen.

Die Prothese weist ein Rotationssystem 6b1 zum Drehen der Hand auf. Eine Steuerung eines Motors des Rotationssystems 6b1 dreht die Prothese entsprechend den Effektorsteuerungssignalen. Außerdem weist die Prothese ein Greifsystem 6b2 mit Motor und Steuerung auf, das den Effektorsteuerungssignalen entsprechende Öffnungs- und Schließbewegungen eines Fingerteils der Hand ausführt. Es sollte erwähnt werden, dass zweckmäßigerweise nicht unbedingt versucht wird, alle Steuerungsdetails aus den neuronalen Daten zu ermitteln. Stattdessen könnte System 5 auch nur die Art der intendierten Bewegung vorhersagen und dann selbstständig die erforderlichen Einzelschritte bestimmen.

Für die Generierung eines funktionellen Feedbacks an das Gehirn sind an dem Fingerteil Drucksensoren 6c angebracht. Deren ermittelte Effektorzustandssignale werden über eine Effektorausgangsleitung 6a2 an das System 5 zurückgegeben. Schließlich ist die Prothese noch von einer Verkleidung umgeben, die zweckmäßigerweise das Aussehen einer menschlichen Hand nachahmt. Anzumerken ist, dass eine Handprothese hier nicht auf das Öffnen und Schließen beschränkt ist, sondern mit technisch fortgeschritteneren Prothesen im Rahmen der Erfindung auch die Durchführung komplexerer Bewegungen möglich ist.

Weiter ist es möglich, mit einer Arm-/Handprothese auf diese Weise sämtliche natürliche Bewegungen eines Armes und/oder einer Hand zu ermöglichen und mit Hilfe geeigneter Sensoren wie Bewegungs-, Abstands- oder Temperatursensoren sowohl die Lageempfindung (oder Propriozeption, also die Kenntnis der Lage des Armes auch bei geschlossenen Augen) als auch den Tastsinn, die Wärmeempfindung etc. des Armes wiederherzustellen.

In der zweiten Gruppe möglicher Effektoren 6 mit besonderer Bedeutung werden eigene Körperteile über funktionelle Elektrostimulation als Effektor 6 angesteuert, sofern nur die neuronale Verbindung zwischen Gehirn und dem Körperteil unterbrochen ist. Dabei werden entweder noch intakte Nervenzellen des Körperteils oder unmittelbar dessen Muskelfasern stimuliert. Ein Feedback kann ebenfalls entweder über noch intakte körpereigene Druck-, Dehnungs-, u.a. Rezeptoren oder, in einer Art Hybrid, mittels unterstützender Sensoren erfolgen, wie oben für den Fall der Prothesensteuerung beschrieben. Ebenso ist auch im Falle von unvollständigen Lähmungen, bei denen noch eine (schwache) Restbewegungsfähigkeit vorhanden ist, die Unterstützung dieser residuellen Bewegungen durch von Motoren getriebenen mechanischen Vorrichtungen denkbar.

Die dritte Gruppe der "virtuellen" Effektoren 6 ist besonders groß. Damit kann ein Computercursor oder eine Menüauswahl, aber auch das Einschalten des Lichts, das Absenden eines Notrufes etc. angesteuert werden. Denkbares Feedback wäre das Anschlagen eines Cursors am Zeilen- oder Seitenende oder eine Fehlermeldung jeder Art.

Besonders interessant ist die Steuerung einer virtuellen Prothese. Dabei wird ein Körperteil dreidimensional auf einem Bildschirm dargestellt und durch neuronale Aktivität des Patienten oder Probanden gesteuert. Durch Interaktion mit einer virtuellen Umgebung kann die Prothese auch anstoßen oder sich erwärmen. Derartige Ereignisse werden per neuronaler Stimulation zurückgemeldet. Insgesamt lässt sich damit eine Prothese trainieren und kalibrieren. Eihe Rückmeldung durch Stimulation oder durch Beobachtung des Effektors kann, das gilt für die Kalibrierung mittels der virtuellen wie für diejenige einer physischen Prothese, die Ansteuerung aufgrund der Lern- und Anpassungsfähigkeit neuronaler Aktivitäten (der neuronalen Plastizität) stark verbessern.

Wenn man solche Steuerungs- und Stimulationsdaten einmal in computerverarbeitbarer Form zur Verfügung hat, ist man natürlich dank des Internets oder ähnlicher Netzwerke nicht mehr auf räumliche Nähe angewiesen. Die anzusteuernden Effektoren müssen sich deshalb nicht in unmittelbarer räumlicher Nähe/Verbindung zu dem den Effektor steuernden Individuum befinden. So könnte eine Prothese oder ein Roboter virtuell dargestellt und gesteuert werden, die tatsächlich an einem anderen Ort stehen. Anwendungen in der Medizin, bei der ein Chirurg aus der Ferne operiert, beim Militär, das Roboter hochpräzise steuern kann, ohne Menschen in Gefahr zu bringen, oder in Verseuchungs- oder sonst unzugänglichen Gebieten wie Kernkraftwerken, der Tiefsee, dem All sind denkbar. Zwar mag auf den ersten Blick die Operation und der Eingriff in den Schädel solche Anwendungen absurd erscheinen lassen. Durch die schonende und verträgliche Multielektrode 1 der Erfindung ist die Akzeptanz aber ganz wesentlich erhöht. Schon heute werden Chips in den Arm implantiert, um so profane Dinge wie den Eintritt in eine Diskothek zu ermöglichen. Es wird deshalb in Zukunft Anwendungsfälle geben, in denen die Risiko/Nutzenabwägung auch ohne eine schwere Krankheit nicht mehr den Einsatz der Multielektrode 1 ausschließt.

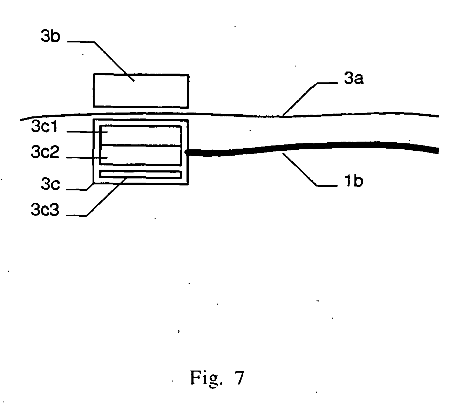

Fig. 7 zeigt eine bevorzugte Ausführungsform der Signalschnittstelle 3. Alternativ könnte als Lösung eine Datenübertragung durch Kabel stattfinden, wie sie bisher in der neurochirurgischen Diagnostik standardmäßig angewendet wird. Eine dauerhafte Kabelverbindung durch die Körperoberfläche birgt allerdings ein erhöhtes Infektionsrisiko und ist auch unter kosmetischen und praktischen Gesichtspunkten wenig attraktiv. Bei der hier beschriebenen bevorzugten Ausführungsform erfolgt die Signalübertragung zwischen Elektrode und Verstärker durch induktive Energieübertragung ohne transkutane Kabelverbindung.

Das drahtlose Signalübertragungssystem 3 ist zweigeteilt in je einen Anteil oberhalb und unterhalb der Hautoberfläche 3a. Von der äußeren Sende/Empfangseinheit außerhalb des Körpers, also hier oberhalb der Hautoberfläche 3a dargestellt, ist nur stellvertretend eine Spule 3b gezeigt. Ob diese äußere Sende/Empfangseinheit lediglich Daten an den Verstärker 4 oder das Rechnersystem 5 drahtlos oder per direkter Kabelverbindung vermittelt oder selbst Verstärker 4 und/oder Rechnersystem 5 teilweise oder vollständig in einem auf der Schädeloberfläche aufsitzenden mobilen Chip enthält, ist wesentlich eine Frage der Komplexität der Anwendung. Derzeit ist zumindest eine kompakte Sende/Empfangseinheit an einen externen Verstärker 4 bzw. ein Rechnersystem 5 über nahezu beliebige Entfernungen (Mobilfunk, Bluetooth, WLAN) technisch ohne weiteres möglich.

Die genannten Übertragungswege können auch für den Datenaustausch mit dem Effektor 6 genutzt werden. Im Falle einer Ansteuerung eines gelähmten natürlichen Körperteils kann eine weitere zweiteilige Signalübertragungsschnittstelle ähnlich der hier beschriebenen in dem jeweiligen Körperteil eingesetzt werden. Da die äußere Sende/Empfangseinheit leicht zugänglich ist, kann sie auch der fortschreitenden Technik angepasst bzw. ausgetauscht werden, ohne dass ein erneuter operativer Eingriff erforderlich wird.

Unterhalb der Hautoberfläche 3a ist als Gegenstück zu der äußeren Sende/Empfangseinheit ein Multifunktionschip 3c als innere Sende/Empfangseinheit eingesetzt. Dieser Multifunktionschip 3c weist eine Empfängereinheit 3c1, eine Sendereinheit 3c2 und optional eine Batterieeinheit 3c3 auf. Über das Kabel 1b werden die Signale von und zu den Elektroden 1c, 1d des Trägers 1a der Sendereinheit 3c2 bzw. der Empfangseinheit 3c 1 zugeführt.

Im Betrieb überträgt die Spule 3b der äußeren Sende/Empfangseinheit Energie und Stimulationssignale induktiv über Hochfrequenzsignale an die Empfangseinheit 3c1. Der Multifunktionschip 3c bestimmt die aufmodulierten Stimulationssignale in aus der Nachrichtentechnik bekannter Weise und leitet sie über das Kabel 1b weiter an die Elektroden 1d des Trägers 1a. Die Energie für die erforderlichen Rechenoperationen von Steuerungseinheiten in dem Multifunktionschip 3c werden aus den Hochfrequenzsignalen bezogen. Alternativ kann Batterie 3c3 bzw. ein Akkumulator induktiv über die Hochfrequenzsignale aufgeladen werden, so dass die Energieversorgung zeitlich von der Übertragung an der Schnittstelle entkoppelt ist. Dabei ist natürlich zwischen Auflade- und Stimulationssignalen zu trennen, etwa durch Zeitfenster oder durch getrennte Frequenzbänder.

Umgekehrt werden Signale der Messelektroden 1c über das Kabel 1b an die Sendereinheit 3c2 übertragen und dort, bevorzugt im Signalband von 402-405 MHz des MICS (Medical Implantable Service Band), auf die Spule 3b oder ein für den Empfang ausgelegtes Gegenstück der nur stellvertretend dargestellten Spule 3b übertragen.

Bisher wurde die Übertragungsschnittstelle so beschrieben, dass die Sendeleistung der Sendeeinheit 3c2 nur bis zu der Spule 3b der äußere Sende/Empfangseinheit reicht. Alternativ könnte die Sendeeinheit 3c2 auch direkt an den Verstärker 4 senden, der möglicherweise sogar nicht auf der Schädeloberfläche aufsitzt. In diesem Fall ist die Energieversorgung des Multifunktionschips 3c entweder durch langlebige Batterien (derzeit technisch nicht zufriedenstellend) oder eine Auflademöglichkeit etwa in der beschriebenen Weise durch Induktion sicherzustellen.

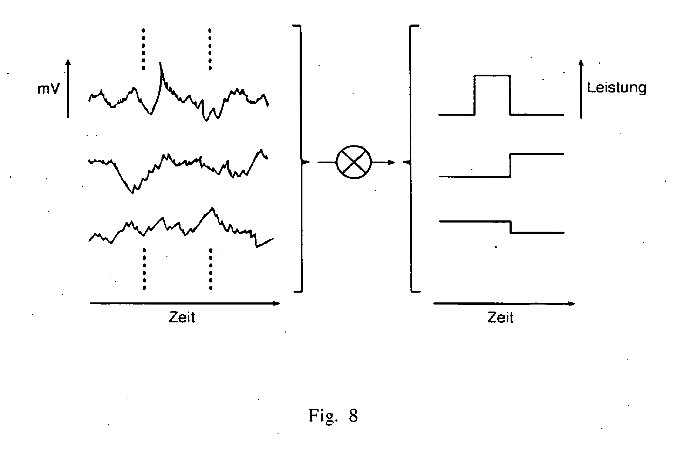

Fig. 8 zeigt eine beispielhafte Schemadarstellung der Umrechnung von neuronalen Signalen in Effektorsteuerungssignale. Auf der linken Seite sind beispielhaft drei Spannungsverläufe von drei Elektroden 1c gezeigt. Diese Spannungssignale werden zunächst als Eingangssignale im Verstärker 4 verstärkt und gefiltert. Die Filterfunktionalität kann auch in dem System 5 lokalisiert sein. Als eine beispielhafte Filtermethode - weitere sind oben im Zusammenhang mit dem Verstärker 4 genannt - werden die Spannungssignale in einem Bandpass gefiltert, anschließend über kleine Zeitfenster gemittelt und in kurze Zeitfenster eingeteilt. Die Aktivität wird dann mittels mathematischer Verfahren ausgewertet, typischerweise durch: (1) Vorverarbeitung der Signale, bspw. a) Filterung (z.B. Tiefpass oder Bandpass), b) Zeit-Frequenzanalyse (z.B. Fourier-Transformation oder Multi-Tapering) und/oder c) Binnung und Mittelung im Zeitbereich; (2) Dekodierung der vorverarbeiteten Signalem bspw. Diskriminantenanalyse (lineare, quadratische oder regularisierte) oder Support-Vektor-Maschine (linear oder radial basis function). Die genannten Methoden sind nicht erschöpfen. Insbesondere zur Dekodierung koninuierlicher Bewegungen sind andere als die genannten Diskriminantenanalyse und Support-Vektor-Machine zu verwenden, beispielsweise lineare Filter oder Kalman-Filter.

Das Ergebnis sind die rechts dargestellten Effektorsteuerungssignale, wobei hier beispielhaft zwei Effektoreinrichtungen, etwa zwei Motoren, und deren aufzubringende Leistung entsprechend ihrer Drehgeschwindigkeit dargestellt sind.

Selbstverständlich ist die Auswertung alles andere als einfach. Dennoch kennt der Fachmann einsatzbereite Verfahren, auch wenn ständig neue hinzukommen oder bestehende verbessert und weiterentwickelt werden. Auch sollte vor dem Einsatz des Systems 5 eine Trainingsphase erfolgen, in welcher der Patient lernt, mit dem System 1-6 umzugehen und umgekehrt das System 5 kalibriert wird.

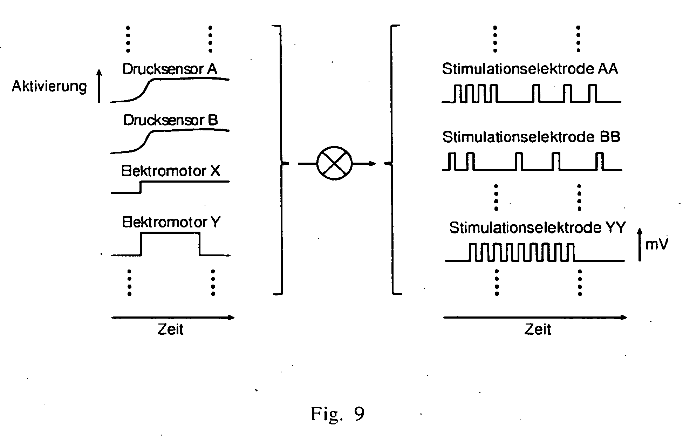

Fig. 9 zeigt den umgekehrten Datenweg in einer beispielhaften Schemadarstellung der Umrechnung von Feedbackdaten eines Effektors in Stimulationssignale für die Elektroden. Links sind Aktivierungsintensitäten verschiedener Drucksensoren und Motoren gegen die Zeit aufgetragen. Diese Aktivierungsintensitäten werden als Effektorzustandssignale dem System 5 übergeben. Dort werden tonische Drucksignale bzw. Motoraktivierung in phasisch-tonisch hochfrequente Stimulationssignale umgerechnet. Diese Stimulationssignale, wie rechts als zeitliche Spannungsverläufe dargestellt, werden jeweils auf eine oder mehrere Elektroden 1d übermittelt, die mit den benachbarten und wegen des gezielten Einsetzens des Trägers 1a auch für den Reiz zuständigen Neuronen in elektromagnetischer Wechselwirkung stehen bzw. diese stimulieren. Bei der Kalibrierung dieses Systems ist die Mitarbeit des Patienten hilfreich, der seine Empfindungen bei Stimulation durch verschiedene Gruppen von Elektroden äußert. Wie bereits im Zusammenhang mit der Umrechnung der Messsignale gesagt, ist auch hier die Entwicklung mit ständiger Verbesserung im Fluss.

### Abschließend sollen noch einmal die Vorteile der Erfindung in einer kurzen Zusammenfassung erläutert werden:

Viele Bereiche des menschlichen Kortex liegen nicht an der Oberfläche, sondern sind in Furchen, Sulci, verborgen. In ihrer Form angepasste Elektroden können hier ohne Gewebe zu verdrängen eingeschoben werden. Besonders relevant kann dies für eine bevorzugte Ausführungsform im somatosensorischen Kortex und Motorkortex sein, weil große Teile des primären motorischen Kortex (welcher eine zentrale Rolle bei der Ausführung von Willkürbewegungen spielt und dessen neuronale Bewegungskodierung am besten untersucht ist) im so genannten *zentralen Sulcus* verborgen liegen. Auf diese Weise werden also Anteile des Kortex, die in der Tiefe der einzelnen Hirnwindungen verborgen liegen (incl. des für die Steuerung willkürlicher Hand- und Aimbewegungen besonders wichtigen Anteils des sog. Brodmann Areals 4) erreichbar.

Eine hier implantierte Elektrode besitzt zudem den Vorteil, das sich der primäre somatosensorische Kortex (welcher propriozeptive Signale empfängt und verarbeitet und somit zur Bewegungswahrnehmung beiträgt) direkt gegenüber des motorischen Kortex befindet, und zwar mit der gleichen somatotopischen Anordnung (d.h. dem zur Bewegungsausführung verantwortlichen Bereich, bspw. der Hand, gegenüber liegt, möglicherweise mit einer Verschiebung, der für die entsprechende Wahrnehmung der Bewegung der Hand verantwortliche Bereich). Eine motorische Prothese, die von einer hier intrasulcal implantierten Elektrode gesteuert wird, ermöglicht somit zusätzlich sensorische Rückmeldungen über die gleiche Elektrode. Damit ist auf einfache und für einen Patienten sehr schonende und verträgliche Weise sogar die Voraussetzung für eine bidirektionale Kommunikation ermöglicht.

### Verwendete Bezugszeichen

| | |
|---|---|
| 1 | Multielektrode |
| 1a | Träger |
| 1b | Kabel |
| 1c | (Ableit)elektroden |
| 1d | (Stimulations)Elektroden |
| 1e | Leiterbahnen |
| 2 | Großhirn / Kortex |
| 2a | somatosensorische Kortex |
| 2b | primärer motorischer Kortex |
| 3 | Signalschnittstelle |
| 3a | Hautoberfläche |
| 3b | Spule der äußeren Sende/Empfangseinheit |
| 3c | Multifunktionschip |
| 3c1 | Empfangseinheit |
| 3c2 | Sendeeinheit |
| 3c3 | Batterie/Akkumulator |
| 4 | Verstärker |
| 5 | System zur Auswertung und zentralen Steuerung |
| 6 | Effektor |
| 6a1 | Effektoreingangsleitung |
| 6a2 | Effektorausgangsleitung |
| 6b1 | Rotationssystem |
| 6b2 | Greifsystem |
| 6c | Drucksensoren |
| 6d | Verkleidung |

## Patentansprüche

1. Sonde zur Datenübertragung zwischen einem Gehirn (2) und einer Datenverarbeitungsvorrichtung (5), wobei die Sonde einen Träger (1a) mit darauf angebrachten Elektroden (1c, 1d) aufweist, die zur Erfassung von neuronaler Aktivität und/oder Übertragung von Stimuli in elektromagnetische Wechselwirkung mit Neuronen des Gehirns (2) gebracht werden können und die mit der Datenverarbeitungseinrichtung (5) koppelbar sind, wobei der Träger (1a) in seiner Form an eine innere Oberfläche des Gehirns (2) derart anpassbar ist, dass er in einem Innenraum eines Sulcus (2c) des Gehirns (2) eingesetzt werden kann,
wobei
der Träger (1a) flexibel ausgestaltet ist und zwei einander gegenüberliegende flache Oberflächen, welche eine Vorderseite und eine Rückseite des Trägers bilden, aufweist und
an den zwei gegenüberliegenden Oberflächen eine erste bzw. zweite Gruppe der Elektroden angebracht ist, wobei die Elektroden als Kontaktflächen ausgestaltet sind, derart, dass die erste Gruppe der Elektroden (1c) mit Neuronen der ersten Seitenwand (2a) des Sulcus (2c) und die zweite Gruppe der Elektroden (1d) mit Neuronen der zweiten Seitenwand (2b) des Sulcus (2c) in elektromagnetische Wechselwirkung kommen können, wobei die Elektroden (1c, 1d) in ihrer Anordnung an die Morphologie der ersten Seitenwand (2a) bzw. der zweiten Seitenwand (2b) anpassbar sind.

2. Sonde nach Anspruch 1, wobei beide Oberflächen des Trägers (1a) eben ausgestaltet sind.

3. Sonde nach einem der vorherigen Ansprüche, wobei die erste Gruppe (1c) Ableitelektroden und die zweite Gruppe (1d) Stimulationselektroden aufweist.

4. Einrichtung zur Datenübertragung zwischen einem Gehirn (2) eines Lebewesens und einer Datenverarbeitungsvorrichtung (5), welche zumindest eine Sonde gemäß einem der vorherigen Ansprüche und eine Auswerteeinrichtung (5) aufweist, welche dafür ausgebildet ist, Signale der Elektroden (1, 1c, 1d) in für die Datenverarbeitungsvorrichtung (5) verarbeitbare Neuronensignale und/oder Ausgangssignale der Datenverarbeitungsvorrichtung (5) in Stimulationssignale für die Elektroden (1, 1c, 1d) umzuwandeln wobei die Auswerteeinrichtung (5) dafür ausgebildet ist, einen ersten Teil der Elektroden (1c) mittels Auslesen der Eingangssignale als Ableitelektroden und einen zweiten Teil der Elektroden (1d) mittels Zuführen der Ausgangssignale als Stimulationselektroden anzusteuern, so dass ein bidirektionaler Datenaustausch ermöglicht ist.

5. Einrichtung nach Anspruch 4, wobei die Auswerteeinrichtung (5) zusätzlich als Effektorsteuerung eines anschließbaren Effektors (6) ausgebildet ist und unter Einbeziehung der Eingangssignale Effektorsteuersignale für den Effektor (6) und/oder unter Einbeziehung von Effektorzustandsignalen des Effektors (6) die Stimulationssignale berechnet.

6. Einrichtung nach Anspruch 5, wobei
- der Effektor eine Prothese (6) ist;
- die Eingangssignale diejenigen von Neuronen im Motorkortex und die Stimulationssignale für Neuronen im somatosensorischen Kortex sind;
- die Effektorsteuersignale Bewegungsparameter (6b1, 6b2) der Prothese (6) beeinflussen und
- die Effektorzustandssignale Stellungs-, Bewegungs- und/oder Zustandsparameter weiterer Sensoren wie Druck-, Berührung-, Abstands-oder Temperatursensoren (6c) sind,
so dass das Gehirn (2) die Bewegung der Prothese (6) steuern kann und unmittelbare somatosensorische Rückmeldung über die Bewegung sowie die Umgebung der Prothese (6) erhält.

7. Einrichtung nach Anspruch 5, wobei
- der Effektor (6) ein Körperteil des Lebewesens ist;
- die Eingangssignale diejenigen von Neuronen im Motorkortex und die Stimulationssignale für Neuronen im somatosensorischen Kortex sind;
- die Effektorsteuersignale Motorneuronen oder Muskelfasern des Körperteils beeinflussen und
- die Effektorzustandssignale Signale von Rezeptoren oder Rezeptorneuronen des Körperteils und/oder Stellungs-, Bewegungs-und/oder Zustandsparameter weiterer Sensoren wie Druck-, Berührungs-, Abstands- oder Temperatursensoren sind,
so dass das Gehirn (2) die Bewegung des Körperteils (6) steuern kann und unmittelbare somatosensorische Rückmeldung über die Bewegung sowie die Umgebung des Körperteils (6) erhält.

8. Einrichtung nach Anspruch 5, wobei
- der Effektor ein Rechner (6) insbesondere mit einer Anzeige ist;
- die Eingangssignale diejenigen von Neuronen im Motorkortex und die Stimulationssignale für Neuronen im somatosensorischen Kortex sind;
- die Effektorsteuersignale virtuelle, insbesondere auf der Anzeige dargestellte Bewegungen oder Funktionen in dem Rechner (6) beeinflussen und
- die Auswerteeinrichtung (5) unter Einbeziehung der virtuellen Bewegung oder der Funktion die Effektorzustandssignale errechnet.

9. Einrichtung nach einem der Ansprüche 4 bis 8, wobei ein Verstärker (4) vorgesehen ist, der für die Verstärkung und Filterung von den Eingangssignalen zu vorverarbeiteten Eingangssignalen und/oder von den Ausgangssignalen zu den Stimulationssignalen ausgebildet ist.

10. Verfahren zur Herstellung einer Sonde für eine Datenübertragung zwischen einem Gehirn (2) und einer Datenverarbeitungsvorrichtung, die folgenden Schritte umfassend:
- Bestimmen der Geometrie eines Sulcus (2c) des Gehirns (2) auf der Basis von strukturellen Bilddaten des Gehirns (2),
- Formen des Trägers (1 a) gemäß der bestimmten Geometrie, um den Träger (1a) in den Sulcus (2c) einsetzbar zu machen,
- Bestimmen der neuronalen Aktivitäten im Bereich des Sulcus (2c) auf der Basis von funktionellen Bilddaten des Gehirns;
- Anordnen von Kontaktflächen (1c, 1d) auf dem Träger (1a) gemäß den bestimmten neuronalen Aktivitäten, derart, dass die Kontaktflächen (1c, 1d) auf dem Träger (1a) Stellen im Bereich, des Sulcus mit signifikanten neuronalen Aktivitäten zugeordnet werden können.

11. Verfahren nach Anspruch 10, wobei die funktionellen Bilddaten die Aktivitäten von Neuronen des Gehirns (2) bei einer Serie von Bewegungsaufgaben repräsentieren, wobei die Bewegungsaufgaben insbesondere Betrachten und Bewegen umfassen.

12. Verfahren nach Anspruch 10 oder 11, wobei der Träger (1a) aus einem flexiblen Material gefertigt wird.

13. Verfahren nach einem der Ansprüche 10 bis 12, wobei eine erste Gruppe (1c) der Elektroden (1e, 1d) für den elektromagnetischen Kontakt mit Neuronen einer ersten Seitenwand (2b) und eine zweite Gruppe (1d) der Elektroden (1c, 1d) für den elektromagnetischen Kontakt mit Neuronen einer zweiten Seitenwand (2a) auf dem Träger angebracht werden.

14. Verfahren zur Datenübertragung zwischen einer Datenverarbeitungsvorrichtung (5) und einem Gehirn (2) eines Lebewesens, bei dem eine Sonde gemäß einem der Ansprüche 1 bis 3 in einem Sulcus eingesetzt ist, wobei die Sonde ausgestaltet ist, Aktivitätssignale durch elektromagnetische Wechselwirkung mit Neuronen des Gehirns (2) zu erfassen und an eine Signalschnittstelle (3) weiterzuleiten, und wobei das Verfahren folgende Schritte aufweist:
- Verstärken und Filtern der Aktivitätssignale der von der Sonde an die Signalschnittstelle (3) weitergeleiteten Aktivitätssignale durch einen Verstärker (4),
- Umwandeln der erfassten Aktivitätssignale in für das Rechnersystem (5) verarbeitbare Neuronensignale und/oder von Ausgangssignalen des Rechnersystems (5) in Stimulationssignale für die Elektroden (1, 1c, 1d),
- Weiterleiten der Aktivitätssignale an das Rechnersystem (5) in Echtzeit;
wobei zur Effektorsteuerung eines angeschlossenen Effektors (6) unter Einbeziehung der Eingangssignale Effektorsteuersignale für den Effektor (6) und/oder unter Einbeziehung von Effektorzustandsignalen des Effektors (6) die Stimulationssignale berechnet werden, wobei der Effektor (6) ein mechanisches Gerät wie Roboter, Roboterarm, Prothese oder ein durch virtuellen Befehl eines Rechners angesteuertes Gerät ist.

## Claims

1. Probe for data transmission between a brain (2) and a data processing device (5), whereby the probe has a support (1a) with electrodes (1c, 1d) fitted thereto which can be brought into electromagnetic interaction with neurons of the brain (2) for detecting neuronal activity and/or transmission of stimuli and which can be coupled with the data processing device (5), whereby the shape of the support (1a) can be adapted to an inner surface of the brain (2) in such a way that it can be inserted into an inner space of a sulcus (2c) of the brain (2),
whereby
the support (1a) is configured flexible and has two opposed flat surfaces which form a front side and a rear side of the support and
a first and respectively second group of the electrodes is fitted on the two opposed surfaces, whereby the electrodes are configured as contact surfaces in such a manner that the first group of the electrodes (1c) can come into electromagnetic interaction with neurons of the first side wall (2a) of the sulcus (2c) and the second group of the electrodes (1d) with neurons of the second side wall (2b) of the sulcus (2c), whereby the arrangement of the electrodes (1c, 1d) can be adapted to the morphology of the first side wall (2a) or of the second side wall (2b).

2. Probe according to claim 1 whereby both surfaces of the support (1a) are configured even.

3. Probe according to one of the preceding claims whereby the first group (1c) has recording electrodes and the second group (1d) stimulation electrodes.

4. Device for data transmission between a brain (2) of a living creature and a data processing device (5) which has at least one probe according to one of the preceding claims and an evaluation unit (5) which is configured to convert signals of the electrodes (1, 1c, 1d) into neuron signals processible for the data processing device (5) and/or output signals of the data processing device (5) into stimulation signals for the electrodes (1, 1c, 1c), whereby the evaluation unit (5) is configured to control a first part of the electrodes (1c) as recording electrodes by reading out the input signals and a second part of the electrodes (1d) as stimulation electrodes by feeding the output signals so that a bidirectional data exchange is enabled.

5. Device according to claim 4 whereby the evaluation unit (5) is additionally configured as effector control of a connectable effector (6) and calculates effector control signals for the effector (6) by integrating input signals and the stimulation signals by integrating effector state signals of the effector (6).

6. Device according to claim 5 whereby
- the effector is a prosthesis (6);
- the input signals are those of neurons in the motor cortex and the stimulation signals for neurons in the somatosensorial cortex;
- the effector control signals influence movement parameters (6b1, 6b2) of the prosthesis and
- the effector state signals are position, movement and/or state parameters of other sensors such as pressure, contact, distance or temperature sensors (6c) so that the brain (2) can control the movement of the prosthesis (6) and receives a direct somatosensorial feedback about the movement as well as the environment of the prosthesis (6).

7. Device according to claim 5 whereby
- the effector (6) is a body part of the living creature;
- the input signals are those of neurons in the motor cortex and the stimulation signals for neurons in the somatosensorial cortex;
- the effector control signals influence motor neurons or muscle fibers of the body part and
- the effector state signals are signals of receptors or receptor neurons of the body part and/or position, movement and/or state parameters of further sensors such as pressure, contact, distance or temperature sensors
so that the brain (2) can control the movement of the body part (6) and receives a direct somatosensorial feedback on the movement as well as the environment of the body part (6).

8. Device according to claim 5 whereby
- the effector is a computer (6) in particular with a display;
- the input signals are those of neurons in the motor cortex and the stimulation signals for neurons in the somatosensorial cortex;
- the effector control signals influence in the computer virtual movements or functions, in particular those represented on the display and
- the evaluation unit (5) calculates the effector state signals by integrating the virtual movement or the function.

9. Device according to one of the claims 4 to 8, whereby an amplifier (4) is provided which is configured for the amplification and filtering of the input signals to pre-processed input signals and/or of the output signals to the stimulation signals.

10. Method for manufacturing a probe for data transmission between a brain (2) and a data processing device that comprises the following steps:
- Determining the geometry of a sulcus (2c) of the brain (2) on the base of structural image data of the brain (2),
- Shaping of the support (1a) according to the determined geometry in order to make the support (1a) insertable in the sulcus (2c),
- Determining the neuronal activities in the region of the sulcus (2c) on the basis of functional image data of the brain,
- Arranging contact surfaces (1c, 1d) on the support (1a) according to the determined neuronal activities in such a manner that the contact surfaces (1c, 1d) on the support (1a) can be assigned to locations in the region of the sulcus with significant neuronal activities.

11. Method according to claim 10 whereby the functional image data represent the activities of neurons of the brain (2) for a series of movement tasks, whereby the movement tasks comprise in particular observation and movement.

12. Method according to claim 10 or 11 whereby the support (1a) is made of a flexible material.

13. Method according to one of the claims 10 to 12 whereby a first group (1c) of the electrodes (1c, 1d) is fitted on the support for the electromagnetic contact with neurons of a first side wall (2b) and a second group (1d) of the electrodes (1c, 1d) for the electromagnetic contact with neurons of a second side wall (2a).

14. Method for data transmission between a data processing device (5) and a brain (2) of a living creature whereby a probe according to one of the claims 1 to 3 is inserted in a sulcus, whereby the probe is configured to detect activity signals by electromagnetic interaction with neurons of the brain (2) and to transmit them to a signal interface (3) and whereby the method has the following steps:
- Amplifying and filtering the activity signals of the activity signals transmitted by the probe to the signal interface (3) by an amplifier (4),
- Converting the detected activity signals into neuron signals processible for the computer system (5) and/or of output signals of the computer system (5) into stimulation signals for the electrodes (1, 1c, 1d),
- Transmitting the activity signals to the computer system (5) in real time,
whereby for the effector control of a connected effector (6) effector control signals for the effector (6) are calculated by integrating the input signals and/or the stimulation signals are calculated by integrating effector state signals of the effector, whereby the effector (6) is a mechanical device such as a robot, a robot arm, a prosthesis or a device controlled a virtual command of a computer.

## Revendications

1. Sonde pour la transmission de données entre un cerveau (2) et un dispositif de traitement de données (5), la sonde présentant un support (1a) avec des électrodes placées dessus (1c, 1d) qui peuvent être amenées en interaction électromagnétique avec des neurones du cerveau (2) pour l'enregistrement d'une activité neuronale et/ou la transmission de stimuli et qui peuvent être reliées au dispositif de traitement de données (5), la forme du support (1a) pouvant être adaptée à une surface interne du cerveau (2) de telle manière qu'il peut être placé dans un espace intérieur d'un sillon (2c) du cerveau,
le support (1a) étant configuré flexible et présentant deux surfaces plates en face l'une de l'autre qui forment un côté antérieur et un côté postérieur du support et
un premier, respectivement un second groupe des électrodes étant installé sur les deux surfaces en face l'une de l'autre, les électrodes étant configurées comme des surfaces de contact de telle manière que le premier groupe des électrodes (1c) peut venir en interaction électromagnétique avec des neurones de la première paroi latérale (2a) du sillon (2c) et le second groupe des électrodes (1d) avec des neurones de la seconde paroi latérale (2b) du sillon (2c), l'arrangement des électrodes (1c, 1d) pouvant être adapté à la morphologie de la première paroi latérale (2a) ou de la seconde paroi latérale (2b).

2. Sonde selon la revendication 1, les deux surfaces du support (1a) étant configurées planes.

3. Sonde selon l'une des revendications précédentes, le premier groupe (1c) présentant des électrodes d'enregistrement et le second groupe (1d) des électrodes de stimulation.

4. Dispositif pour la transmission de données entre un cerveau (2) d'un être vivant et un dispositif de traitement de données (5) qui présente au moins une sonde selon l'une des revendications précédentes et un dispositif d'évaluation (5) qui est configuré pour transformer des signaux des électrodes (1, 1c, 1d) en signaux de neurones pouvant être traités pour le dispositif de traitement de données (5) et/ou des signaux de sortie du dispositif de traitement de données (5) en signaux de stimulation pour les électrodes (1, 1c, 1d), le dispositif d'évaluation (5) étant configuré pour contrôler une première partie des électrodes (1c) comme électrodes d'enregistrement au moyen de l'extraction des signaux d'entrée et une seconde partie des électrodes (1d) comme électrodes de stimulation au moyen de l'amenée des signaux de sortie de telle manière qu'un échange de données bidirectionnel soit rendu possible.

5. Dispositif selon la revendication 4, le dispositif d'évaluation (5) étant configuré de plus comme une commande d'effecteur d'un effecteur pouvant être branché (6) et calculant les signaux de commande d'effecteur pour l'effecteur (6) en intégrant les signaux d'entrée et/ou les signaux de stimulation en intégrant les signaux d'état d'effecteur de l'effecteur (6).

6. Dispositif selon la revendication 5,
- l'effecteur étant une prothèse (6) ;
- les signaux d'entrée étant ceux des neurones du cortex moteur et les signaux de stimulation étant pour des neurones dans le cortex somatosensoriel;
- les signaux de commande d'effecteur influençant des paramètres de mouvement (6b1, 6b2) de la prothèse (6) et
- les signaux d'état d'effecteur étant des paramètres de position, de mouvement et/ou d'état d'autres capteurs comme les capteurs de pression, de contact, de distance ou de température
si bien que le cerveau (2) peut commander le mouvement de la prothèse (6) et reçoit un retour somatosensoriel direct par le mouvement ou l'environnement de la prothèse (6).

7. Dispositif selon la revendication 5,
- l'effecteur (6) étant une partie du corps de l'être vivant ;
- les signaux d'entrée étant ceux des neurones dans le cortex moteur et les signaux de stimulation pour les neurones étant dans le cortex somatosensoriel ;
- les signaux de commande de l'effecteur influençant les neurones moteurs ou les fibres musculaires de la partie du corps et
- les signaux d'état de l'effecteur étant des signaux de récepteurs ou des neurones récepteurs de la partie du corps et/ou des paramètres de position, de mouvement et/ou d'état d'autres capteurs comme les capteurs de pression, de contact, de distance ou de température
si bien que le cerveau (2) peut commander le mouvement de la partie du corps (6) et reçoit un retour somatosensoriel direct par le mouvement ou l'environnement de la partie du corps (6).

8. Dispositif selon la revendication 5,
- l'effecteur étant un ordinateur (6), en particulier avec un affichage ;
- les signaux d'entrée étant ceux des neurones dans le cortex moteur et les signaux de stimulation pour des neurones dans le cortex somatosensoriel ;
- les signaux de commande de l'effecteur influençant dans l'ordinateur (6) des mouvements ou des fonctions virtuelles, en particulier représentées sur l'affichage, et
- le dispositif d'évaluation (5) calculant les signaux d'état de l'effecteur en intégrant le mouvement ou la fonction virtuelle.

9. Dispositif selon l'une des revendications 4 à 8, un amplificateur (4) étant prévu qui est configuré pour l'amplification et le filtrage des signaux d'entrée en signaux d'entrée prétraités et/ou des signaux de sortie en signaux de stimulation.

10. Procédé pour la fabrication d'une sonde pour une transmission de données entre un cerveau (2) et un dispositif de traitement de données comprenant les étapes suivantes :
- Détermination de la géométrie d'un sillon (2c) du cerveau (2) sur la base de données d'image structurelles du cerveau (2),
- Formation du support (1a) selon la géométrie déterminée pour pouvoir rendre le support (1a) utilisable dans le sillon (2c),
- Détermination des activités neuronales dans la zone du sillon (2c) sur la base de données d'image fonctionnelles du cerveau ;
- Placement de surfaces de contact (1c, 1d) sur le support (1a) selon les activités neuronales déterminées de telle manière que les surfaces de contact (1c, 1d) sur le support (1a) peuvent être assignées à des endroits dans la zone du sillon avec des activités neuronales importantes.

11. Procédé selon la revendication 10, les données d'image fonctionnelles représentant les activités des neurones du cerveau (2) pour une série de tâches de mouvement, les tâches de mouvement comprenant en particulier l'observation et le déplacement.

12. Procédé selon la revendication 10 ou 11, le support (1a) étant fabriqué en un matériau flexible.

13. Procédé selon l'une des revendications 10 à 12, un premier groupe (1c) des électrodes (1c, 1d) étant monté sur le support pour le contact électromagnétique avec des neurones d'une première paroi latérale (2b) et un second groupe (1d) des électrodes (1c, 1d) pour le contact électromagnétique avec des neurones d'une seconde paroi latérale (2a).

14. Procédé pour la transmission de données entre un dispositif de traitement de données (5) et un cerveau (2) d'un être vivant pour lequel une sonde selon l'une des revendications 1 à 3 est mise en place dans un sillon, la sonde étant configurée pour enregistrer des signaux d'activité par interaction électromagnétique avec des neurones du cerveau (2) et pour les transmettre à une interface de signal (3) et le procédé comprenant les étapes suivantes :
- Amplification et filtrage des signaux d'activité des signaux d'activité transmis par la sonde à l'interface de signal (3) par un amplificateur (4),
- Transformation des signaux d'activité enregistrés en signaux de neurones pouvant être traités pour le système d'ordinateur (5) et/ou de signaux de sortie du système d'ordinateur (5) en signaux de stimulation pour les électrodes (1, 1c, 1d),
- Transmission des signaux d'activité au système d'ordinateur (5) en temps réel,
pour la commande de l'effecteur d'un effecteur branché (6), des signaux de commande d'effecteur pour l'effecteur (6) étant calculés en intégrant les signaux d'entrée et/ou les signaux de stimulation étant calculés en intégrant des signaux d'état d'effecteur de l'effecteur (6), l'effecteur (6) étant un appareil mécanique comme un robot, un bras de robot, une prothèse ou un appareil contrôlé par la commande virtuelle d'un ordinateur.
